# EUROPEAN PATENT APPLICATION

(11) **EP 2 422 722 A1**
(43) Date of publication of application: **29.02.2012**
(21) Application number: 11006753.5
(22) Date of filing: 18.08.2011
(51) Int. Cl.: A61B 17/34, A61B 19/00

(54) **Surgical apparatus**

(30) Priority: 31.08.2010 US 872208
(71) Applicant: Elekta AB (PUBL), 103 93 Stockholm (SE)
(72) Inventor: Alksnis, Ivacs, 142 64 Trangsund (SE)
(74) Representative: Hall, Christopher David

(57) **Abstract**

A guide for a surgical instrument has a support and an instrument holder. One of the support and the instrument holder has a partial spherical surface, and the other has three of more contact points arranged to be coincident with the surface of a sphere having substantially the same radius as the spherical surface. In use, the spherical surface and the contact points are placed in contact with each other, and the orientation of a surgical instrument can be adjusted by relative movement of the contact points and the spherical surface.

## Description

### FIELD OF THE INVENTION

The present invention relates to the field of surgery, and particularly to apparatus and methods suitable for use in neurosurgery.

### BACKGROUND ART

In the field of neurosurgery, it is often necessary to insert an instrument into the patient's brain tissue in order to perform a surgical step at an internal site. A wide range of examples exist; excess fluid can be aspirated, malignant or malfunctioning regions can be cauterised, or regions can be stimulated using temporary, seml-permanent or permanently implanted probes. In all these (and other) cases, an instrument of some description needs to be inserted so that its operative region - usually the tlp - is accurately positioned at a specific location within the cranium.

To allow access, an incision is made in order to reveal the skull, and an aperture is cut in the bone at a suitable location to reveal the soft tissue beneath. It then remains only to align the instrument, which usually has an elongate aspect, so that it can be guided in a straight line towards the specific location. This is a sensitive task that requires a high degree of accuracy in order to avoid collateral damage to other structures within the brain.

Generally, an image of the brain is taken prior to surgery (e.g. by MRI), and an insertion route is planned which avoids internal neurological structures, and the like. During surgery, however, it may occasionally be necessary to alter this planned route if, for example, blood vessels are found which obstruct the intended route. The "centre of arc" principle provides a solution to this problem, allowing alteration of the planned trajectory through the brain whilst ensuring the altered trajectory passes through the target position. Essentially, the surgical instrument is constrained to move in an arc which has at its centre the surgical target. Thus the surgical instrument always points towards the target.

Several systems for stereotactic surgery make use of this principle. Examples include the Leksell Stereotactic System®, as well as the systems from Micromar and Radlonlcs.

While giving the desired flexibility to adjust the trajectory without leaving the target, conventional centre of arc mechanics generally requires a large and bulky system comprising an arc (or arcs) that can pivot around two rotational axes, each axis passing through the target point. These somewhat bulky systems may limit the access for the surgeon and they may also limit the choice of construction material to lightweight materials not always suited for high-precision manufacturing.

### SUMMARY OF THE INVENTION

Imaging techniques (e.g. MRI) are constantly improving, allowing more and more diffuse structures to be detected and identified. As a result, the need for flexibility to change the trajectory around the entire head is decreasing. It is unusual to change the point of entry into the head on a large scale. Typically, the trajectory can be sufficiently altered without departing from the drilled hole in the skull bone, and thus it would be enough to allow for an adjustment within the drill hole diameter (typically around 14 mm).

The invention is a mechanical principle that allows adjustment of the trajectory without altering target position. The principle is operative only in a local area around the nominal trajectory (according to plan) and typically it will allow for adjustment within the drilled hole for the surgery but not far outside the same.

A first embodiment of the present invention provides a guide for a surgical instrument, comprising an instrument holder for holding the surgical instrument and a support for the instrument holder. One of the instrument holder and the support comprises a bearing presenting at least three contact points that are arranged to be coincident with the surface of a sphere. The other of the instrument holder and the support comprises a partial spherical surface having substantially the same radius as the sphere. In use, the contact points are placed in contact with the spherical surface, the orientation of said surgical instrument thereby being adjustable by relative movement of the contact points and the spherical surface.

In one embodiment, the support comprises the bearing and the instrument holder comprises the partial spherical surface. In that case, the bearing may comprise a collar, a surface of which presents the contact points. The collar may be one end of a cylindrical member, an end surface of which presents the contact points.

In another embodiment, the bearing comprises a partial spherical surface having substantially the same radius as the sphere.

The at least three contact points may be presented by one or more continuous lines of contact, such as a circular edge or surface.

The guide may further comprise an interface coupled to the support, for attachment to a patient. The position of the support may be adjustable, relative to the interface, in a plane substantially tangential to a surface of the patient, and/or In a direction substantially perpendicular to a surface of the patient.

In further embodiments of the present invention, there is provided a surgical apparatus, comprising a guide as described above, and an elongate surgical instrument for insertion into the instrument holder. The instrument has a stop co-operating with a part of the instrument holder, defining a length for an operative part of the surgical instrument that corresponds to a radius of the sphere.

The present invention also comprises a method of performing surgery as set out in the claims appended hereto.

Embodiments of the present invention therefore provide a small and lightweight system, which nonetheless preserve the flexibility and simplicity to change the trajectory of a surgical apparatus within the burr hole without altering the target point and without need for re-planning.

### BRIEF DESCRIPTION OF THE DRAWINGS

An embodiment of the present invention will now be described by way of example, with reference to the accompanying figures in which;
Figure 1 shows the application to a patient of apparatus according to embodiments of the present invention;
Figure 2 is a close-up view of apparatus according to embodiments of the present invention; and
Figures 3a to 3c show schematic views of the apparatus and its mode of operation.

### DETAILED DESCRIPTION OF THE EMBODIMENTS

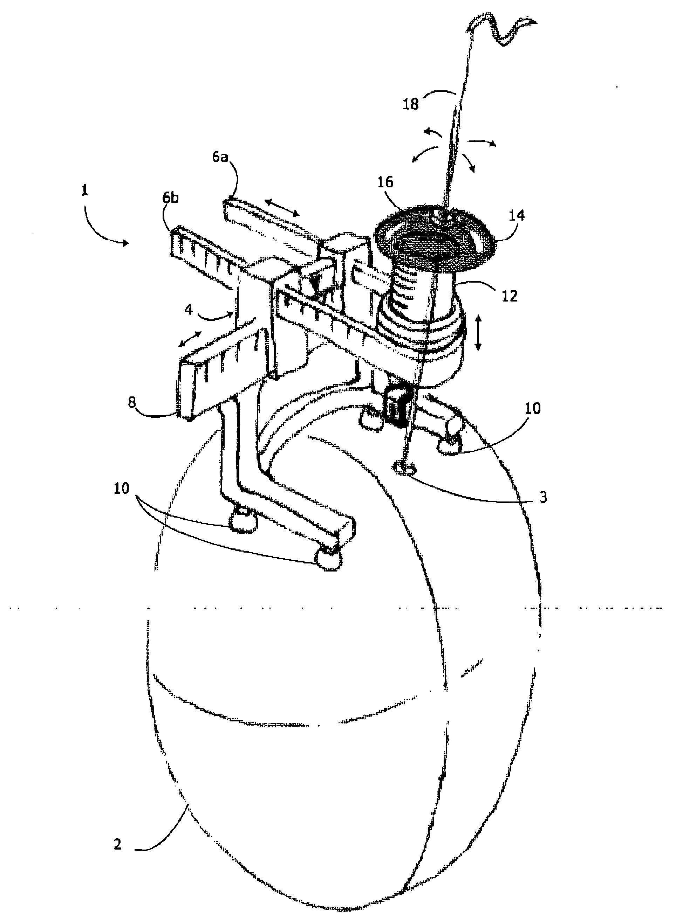

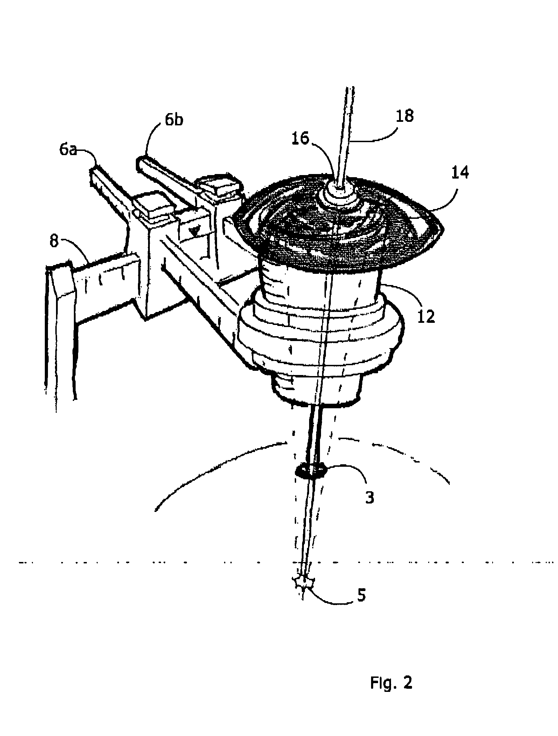

Figure 1 shows a surgical apparatus 1 according to embodiments of the present invention. Figure 2 shows the surgical apparatus whilst performing an operation on a target 5.

The apparatus is most suitable for use in neurosurgery, and so is illustrated in use on the head 2 of a patient. However, the apparatus 1 may also have application in other areas of surgery. As is conventional for neurosurgery, to allow access to the brain an incision is made in order to reveal the skull, and an opening 3 is drilled in the bone at a suitable location to reveal the soft tissue beneath.

An instrument guide, indicated generally by the reference numeral 4, is connected to the patient at four attachment points 10 around the opening 3. It is important that the guide 4 be rigidly fixed to the patient to ensure an accurate frame of reference in which to insert surgical instruments. For that reason, the attachment points 10 usually comprise screws that are physically screwed into the patient's skull. However, alternative attachment points will be familiar to those skilled in the art and are suitable for use with the present invention provided they convey sufficient rigidity between the apparatus and the patient. Any number of attachment points equal to or greater than three should be sufficient.

The guide 4 has a pair of parallel arms 6a, 6b coupled, directly or indirectly, to an orthogonal support member 8. The arms 6a, 6b themselves support a cylindrical collar 12 and part-spherical instrument holder 14 (to be described in greater detail below). In operation, the parallel arms 6a, 6b may be slid along the support member 8 to allow movement of the collar and instrument holder in that direction. The parallel arms themselves can extend in a direction orthogonal to the support member, to allow movement of the collar and instrument holder in that orthogonal direction. Thus, the position of the collar and instrument holder can be adjusted in a plane that is substantially tangential to the surface of the patient 2, for alignment with the burr hole 3. Once a suitable position has been determined, the positions of the arms 6a, 6b can be locked in place.

As previously mentioned, the arms 6a, 6b together support a cylindrical collar 12. In the illustrated embodiment, the arms 6a, 6b form part of the same body: a bar which is bent backwards on itself. The collar 12 is supported on the inside of the bend linking the two arms. The collar 12 is a hollow, cylindrical object with a circular cross-section. An upper surface of the collar (i.e. the surface that is furthest removed from the attachment points 10 and from the patient 2 in general) can be moved in a radial direction away from the patient. In one embodiment, this is achieved by adjusting the position of the collar 12 relative to the arms 6a, 6b. However, in another embodiment the parallel arms 6a, 6b may be able to move in that direction. Alternative means of achieving radial displacement of the upper surface from the patient may be employed by those skilled in the art without inventive activity.

An instrument holder 14 rests on the upper surface of the collar 12. The holder comprises a curved surface which, in the illustrated embodiment, forms part of a sphere. In use, the curved surface rests on the upper surface of the collar 12, and is moveable thereon. That is, the curved surface can effectively "slide" over the (circular) upper surface, remaining in contact therewith as it does so.

An aperture 16 is formed in the curved contact surface of the holder, through which an elongate surgical instrument 18 can be inserted. The aperture 16 is such that the connection between the holder 14 and the instrument 18, when inserted, is rigid. The-only movement that is allowed by the coupling between the holder 14 and the instrument 18 is a radial movement towards and away from the geometrical centre of the spherical surface. The instrument 18 must not be allowed to deviate from that direction. Separate connectors between the holder 14 and the instrument 18 may be provided for that purpose, although these are not illustrated for clarity.

It will be apparent to those skilled in the art that the collar 12 can take various forms. Its primary function is to support the instrument holder 14 on an upper surface, and allow movement of the holder over that upper surface. Thus, in fact an upper surface with at least three support points on the surface of a (hypothetical) sphere would be sufficient. As used herein, the upper surface is taken to mean any collection of three or more points on the surface of a sphere. A particularly useful shape meeting those requirements is a circle, or ring, and this has the added benefit of providing an outer boundary to movement of the instrument inserted through the aperture 16. One form which provides a ring-shaped upper surface with high structural rigidity is a cylinder, as shown in the illustrated embodiment.

In order to provide a centre of arc, the apparatus must be calibrated for use with each patient.

First, attachment fixings 10 are inserted into the patient 2 in the region of the planned insertion point. Generally, the fixings are screwed into place to achieve a sufficiently rigid coupling between the patient and the apparatus 4. With the fixings 10 in place, an image is taken of the patient. This may be achieved through magnetic resonance imaging or any other suitable imaging technique.

A surgeon then examines the image, locates the target 5 and plans an insertion route to the target. The insertion route is planned to avoid sensitive neurological structures, major blood vessels, etc. The fixings 10 act as reference points, or fiducials, to aid in the location of the target.

In the operating theatre, the apparatus 4 is attached to the fixings 10. Thanks to the fiducials provided by the fixing points, the location of the apparatus is accurately known with respect to the location of the target.

As is conventional for neurosurgery, an incision is made in order to reveal the skull, and an aperture 3 is drilled in the bone at a suitable location to reveal the soft tissue beneath.

The collar 12 and instrument holder 14 are now aligned with the hole 3, to allow an instrument 18 to be inserted therethrough to the target. This may be achieved through appropriate movement and extension of the arms 6a, 6b; however, alternative methods will be familiar to those skilled in the art. Ideally, the central axis of the cylindrical collar 12 should pass through the centre of the hole 3 and through the target. Once this alignment is achieved, the positions of the arms 6a, 6b are locked in place.

The height of the collar 12, and particularly its upper surface, must now be set ("height" being equivalent to the radial distance from the target). The curved surface of the instrument holder 14 forms part of a sphere. In order for movement of the holder 14 over the upper surface of the collar to provide a centre of arc at the target, the distance of the upper surface from the target should be equal to the radius of that sphere. The height of the collar can be set accordingly using any one of the methods discussed above.

It may become apparent during the course of surgery that an alternative route is required to the target, for example in order to avoid a structure that was not evident in the acquired imaging data. Such an alternative route may be planned by shifting the holder 14 over the upper surface of the collar 12, keeping the instrument aperture 16 within the bounds of the collar 12 upper surface. The adapted route therefore corresponds to a locus drawn between the aperture 16 and the target. Thanks to the interaction of the spherical surface of the holder 14 and the upper surface of the collar 12, the orientation of the aperture 16 is maintained in a direction towards the target. Once the appropriate route has been found, the holder 14 can be locked in place to fix the route, for example by damping the holder 14 against the collar 12 using tightening screws or a ring pressed against the holder 14 from above (not illustrated). Alternatively, the holder 14 may be fixed to some other external reference point. Various means of fixing the position of the holder 14 relative to the collar 12 will be apparent to those skilled in the art without departing from the scope of the invention as defined by the claims appended hereto. The instrument 18 can then be inserted through the aperture 16, through the collar 12, and through the hole 3 towards the target region inside the patient.

It is equally important that the instrument 18 extends the appropriate distance to the target. For that reason, the surgical instrument 18 may comprise a "stop" (not illustrated) which has a wider diameter than the aperture 16 and prevents the instrument from extending too far into the patient. The stop is positioned on the instrument 18 such that the length of the instrument between the stop (or, more accurately, the part of the instrument coincident with the curved contact surface of the holder 14) and the operative region of the instrument (usually the tip) is equal to the radius of the curved contact surface. Once inserted up to the stop, therefore, the surgeon can be confident that the operative region of the instrument 18 is at the target at the centre of the arc.

The instrument holder 14 and the instrument 18 are therefore complementary in design: the radius of curvature of the instrument holder 14 equal to the length of the instrument 18 below the stopping point.

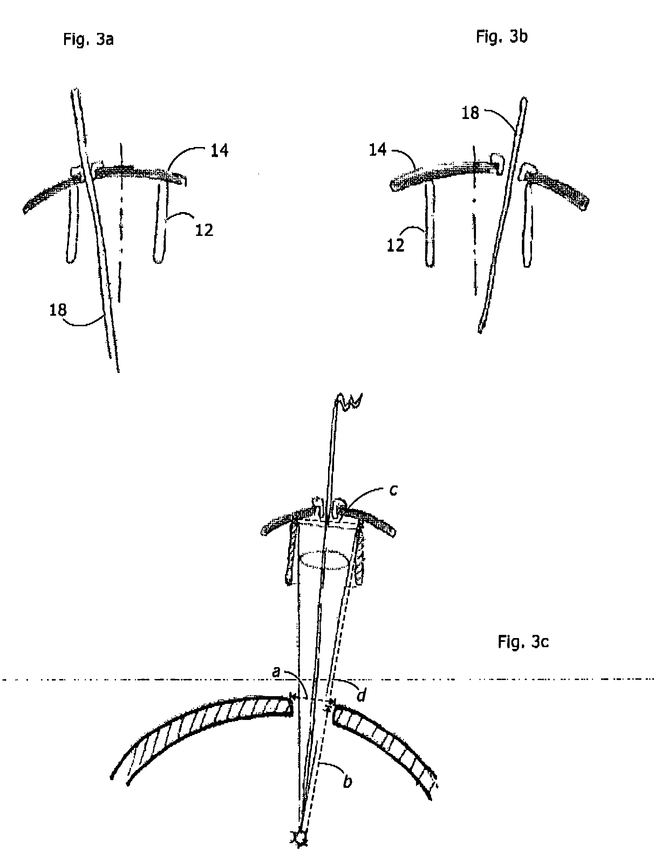

A further consideration may come into play in the design of the cylindrical collar 12. As can be seen from Figures 3a and 3b, the solid angle of possible routes towards the target is limited by the diameter of the collar 12. The instrument has only a local arc of possible routes; it cannot move further along the arc as it is blocked by the fixed position of the collar.

However, there is another limitation to the movement of the instrument, provided by the diameter of the burr hole 3 (assuming the surgeon does not wish to go to the added trouble of widening the hole 3). As with the collar 12, the instrument 18 cannot pass along a route outside the limits of the hole 3. For that reason, the diameter of the collar 12 may be carefully chosen to give the maximum possible range of possible routes to the instrument. This can be achieved by ensuring that the ratio of the collar diameter to the distance of the upper surface from the target is equal to the ratio of the burr hole diameter (usually 14 mm) to the distance of the target from the burr hole. However, the former ratio may also be greater than the latter. In other words, using the dimensions as shown in Figure 3c, *c*/*d* ≥ *a*/*b*. If the opposite is true, then the collar width may suggest alternative routes that are not possible due to the slender width of the burr hole 3.

It will also be apparent to those skilled in the art that there are alternative structures which operate using exactly the same principles as described above. For example, the illustrated embodiment is one in which a support, in the form of a collar, presents a circular upper surface and the instrument holder has a partial spherical surface. In use, the spherical surface moves over the circular surface. The description above broadens this structure by stating that the circular upper surface in fact only requires three or more contact points arranged to be coincident with the surface of a sphere. The position of the contact points may be adjustable using micrometer screws. For example, three points arranged at the points of an equilateral triangle would also be sufficient to support the spherical surface of the instrument holder, allowing relative movement of the instrument holder centred at the surgical target. Equally, the upper surface of the collar 12 may in fact comprise its own partial spherical curved surface, over which the spherical surface of the instrument holder can move.

Once this principle is understood, it will be clear to those skilled in the art that an equivalent structure is one in which the support comprises a partial spherical surface, and the instrument holder comprises three of more contact points arranged to be coincident with the surface of a sphere. The contact points of the instrument holder may then run over the partial spherical surface of the support. Similar principles apply to this embodiment: the three or more contact points of the instrument holder may be presented by one or more continuous contact lines, such as a circle, or a partial spherical surface.

There is thus described apparatus and a method for performing surgery, and particularly neurosurgery, in which a local centre of arc is provided such that a surgeon can change a planned insertion route to an internal target in a patient within the burr hole without the need for replanning. The apparatus is light and simple to use in comparison to prior art systems.

It will of course be understood that many variations may be made to the above-described embodiment without departing from the scope of the present invention.

## Claims

1. A guide for a surgical instrument, comprising:
an instrument holder for holding the surgical instrument; and
a support for the instrument holder;
wherein;
one of the instrument holder and the support comprises a bearing presenting at least three contact points that are arranged to be coincident with the surface of a sphere;
the other of the instrument holder and the support comprises a partial spherical surface having substantially the same radius as the sphere;
and wherein, in use, the contact points are placed in contact with the spherical surface, the orientation of said surgical instrument thereby being adjustable by relative movement of the contact points and the spherical surface.

2. A guide according to claim 1 in which the support comprises the bearing and the instrument holder comprises the partial spherical surface.

3. A guide according to claim 2 in which the bearing comprises a collar, a surface of which presents the contact points.

4. A guide according to claim 3 in which the collar is one end of a cylindrical member, an end surface of which presents the contact point.

5. A guide according to any one of the preceding claims, in which the bearing comprises a partial spherical surface having substantially the same radius as the sphere.

6. A guide according to any one of the preceding claims, in which the at least three contact points are presented by one or more continuous lines of contact.

7. The guide according to claim any one of the preceding claims, further comprising an interface coupled to the support, for attachment to a patient.

8. The guide according to claim 7, wherein the position of the support can be adjusted, relative to the interface, in a plane substantially tangential to a surface of the patient.

9. The guide according to claim 7 or 8, wherein the position of the support can be adjusted, relative to the interface, in a direction substantially perpendicular to a surface of the patient.

10. A surgical apparatus, comprising:
a guide as claimed in any one of the preceding claims; and
an elongate surgical instrument for insertion into the instrument holder, the instrument having a stop co-operating with a part of the instrument holder, defining a length for an operative part of the surgical instrument that corresponds to a radius of the sphere.
